# EUROPEAN PATENT APPLICATION

(11) **EP 4 831 190 A1**
(43) Date of publication of application: **28.10.2026**
(21) Application number: 24905720.9
(22) Date of filing: 26.09.2024
(51) Int. Cl.: C07K 7/08, A61K 9/06, A61K 47/42, A61K 35/28, A61K 45/00, A61P 17/00, A61P 17/18, A61P 17/02, A61P 17/10, A61P 27/02, A61P 25/00, A61P 15/00, A61P 15/08, A61P 15/02, A61P 1/16, A61P 13/12, A61P 37/06, A61P 19/02, A61P 29/00, A61P 1/02

(54) **POLYPEPTIDE AND USE THEREOF**

(30) Priority: 19.12.2023 CN 202311750996
(71) Applicant: Shanghai Jiaotong University School of Medicine, Shanghai 200025 (CN)
(72) Inventor: GAO, Xiaoling, Shanghai 200025 (CN); HUANG, Meng, Shanghai 200025 (CN); SONG, Qingxiang, Shanghai 200025 (CN); HUANG, Yukun, Shanghai 200025 (CN)
(74) Representative: Dai, Simin
(86) International application number: PCT/CN2024/121264
(87) International publication number: WO 2025/130230

(57) **Abstract**

Provided are a polypeptide and the use thereof. The polypeptide has a sequence of KSLSLSLGPASLSLSLK. The polypeptide is prepared into a responsive polypeptide hydrogel, which encapsulates extracellular vesicles to form an extracellular vesicle hydrogel, thereby improving the retention of the extracellular vesicles at an administration site, controlling the release of the extracellular vesicles from the hydrogel, and further improving the utilization efficiency of the extracellular vesicles. The polypeptide hydrogel has a good biocompatibility, and the structure is less affected by changes in physiological or pathological environments. The polypeptide hydrogel is universal for different diseases and different administration sites, promotes the clinical transformation application of the extracellular vesicles, and is also expected to become a universal drug carrier for improving the utilization efficiency of delivered drugs.

## Description

### TECHNICAL FIELD

The present disclosure relates to the field of biomedicine, and in particular to the field of drug delivery systems.

### BACKGROUND

Extracellular vesicles (EVs), as an emerging cell-free therapeutic approach, hold great potential for the treatment of serious diseases. Owing to their stable lipid bilayer membrane structure, extracellular vesicles can transport and deliver bioactive molecules such as proteins and RNA between cells, thereby mediating intercellular signaling and exerting regulatory effects on recipient cells. After release, extracellular vesicles may interact with the extracellular matrix and neighboring cells within the microenvironment to exert paracrine effects, or may be transported by body fluids to distant target cells to exert telecrine effects. Due to their functions in biological information transfer and regulation of target cells, extracellular vesicles play an important role in physiological and pathological processes such as antigen presentation, tumor growth and migration, and tissue repair, thereby demonstrating potential for clinical applications. In recent years, extracellular vesicles have garnered increasing attention with growing applications in disease treatment and drug delivery. Natural stem cell-derived extracellular vesicles have demonstrated great potential in the treatment of cancer, tissue repair, inflammatory diseases, and neurological disorders. Engineered extracellular vesicles have also attracted extensive interest as delivery carriers for biomolecular therapeutics. However, short half-life, rapid clearance, and low retention at the administration site pose significant challenges to the clinical translation of extracellular vesicles. The method of administering extracellular vesicles varies depending on the target organ of the disease. A search of clinical studies on the ClinicalTrails.gov website has revealed that exoSTING, an exosome (a type of extracellular vesicle) developed by Codiak BioSciences, is administered via intratumoral injection (NCT04592484) for the treatment of solid tumors; a study using extracellular vesicles for antiplatelet therapy in acute myocardial infarction involves oral administration (NCT02931045); a clinical study on mesenchymal stem cell (MSC)-derived exosomes for the treatment of dry eye syndrome (NCT04213248) involves direct ocular administration by dispersing exosomes in artificial tears; a clinical study on MSC-derived exosomes for the treatment of acute stroke (NCT03384433) involves direct intracranial injection of exosomes; and a clinical study on MSC-derived extracellular vesicles for the treatment of COVID-19 (NCT04276987) involves administration via nebulization of extracellular vesicles followed by active inhalation by patients.

It can thus be seen that MSC-derived extracellular vesicles in current clinical studies are primarily administered locally. For neurological disorders, treatment typically involves a single local injection, while there is a lack of research on chronic diseases requiring long-term and repeated administration, which may be attributed to two factors. First, compared to synthetic drugs, extracellular vesicles are characterized by lengthy preparation, multiple processing steps, and high costs, making them expensive and difficult to support long-term intensive administration. Second, extracellular vesicle-based therapeutics face challenges such as short half-life and rapid clearance in practical applications. Due to opsonization and clearance by the mononuclear phagocyte system, intravenously administered extracellular vesicles have a half-life of only 1 to 6 hours, and the cleared extracellular vesicles accumulate in the spleen and liver. Therefore, rapid clearance by the innate immune system limits the application of extracellular vesicles as effective therapeutics. In summary, extracellular vesicles require more targeted delivery to reduce waste during administration or *in vivo* and to improve delivery efficiency. Depending on the target site of the disease, it is necessary to conduct research and development on delivery formulations tailored to the characteristics of extracellular vesicles in order to meet the requirements for more efficient clinical translation of extracellular vesicles.

To address the aforementioned delivery issues associated with extracellular vesicles, researchers have developed novel delivery strategies using scaffold materials, by combining gel materials with extracellular vesicles followed by local injection to enhance the retention of extracellular vesicles at the lesion site and maintain their stability, thereby improving the delivery efficiency of extracellular vesicles. Hydrogels, which are three-dimensional structures with a certain viscosity formed by the swelling of polymer materials in water, have been widely used for sustained drug release. However, conventional hydrogels used as local sustained-release systems primarily rely on drug diffusion and material degradation for drug release, which is slow and lacks controllability, often failing to achieve the expected improvement in bioavailability. Since there are significant differences between the microenvironments of normal tissues and diseased sites in the human body, environmentally responsive drug delivery systems prepared according to the local physiological or pathological microenvironment at the administration site have garnered widespread attention in recent years. Responsive hydrogels can sense and respond to the surrounding microenvironment (*e.g.*, temperature, pH, light, ions, enzymes, pressure, electric fields, and magnetic fields), thereby inducing changes such as assembly or degradation of the gel network structure. Although environmentally responsive hydrogels can control drug release through degradation of their network triggered by external stimuli, their molecular structures must be specifically designed according to the microenvironment of different administration sites or therapeutic targets, with subtle changes in the physiological or pathological environment potentially affecting drug release performance, thereby limiting their applicability.

Accordingly, there is an urgent need for a more efficient drug delivery carrier to address the delivery issues associated with extracellular vesicles, improve bioavailability, and better realize the therapeutic potential of MSC-EVs in disease treatment.

### SUMMARY

An objective of the present disclosure is to provide a polypeptide, a polypeptide hydrogel, a responsive extracellular vesicle hydrogel, and the use thereof, wherein the polypeptide hydrogel serves as a drug carrier to improve delivery efficiency, particularly addressing the issue of low utilization efficiency of extracellular vesicles.

To achieve the aforementioned objective, the present disclosure provides the following technical solutions:
A polypeptide, the polypeptide has the sequence of KSLSLSLGPASLSLSLK (referred to as the KPK polypeptide).

A polypeptide hydrogel formed by mixing an isotonic solution of the polypeptide with a salt solution. The polypeptide hydrogel is formed by spontaneous self-assembly of the KPK polypeptide into nanofibers in the presence of salt ions or human tissue fluid, followed by further crosslinking to form a porous network structure.

The concentration of the polypeptide hydrogel is 0.1 w/v% to 50 w/v%; preferably 0.1 w/v% to 10 w/v%; more preferably 0.1 w/v% to 2 w/v%; and even more preferably 1 w/v%.

As a preferred embodiment, the isotonic solution comprises an aqueous solution of a monosaccharide or disaccharide, the isotonic solution of the monosaccharide or disaccharide comprises one or more of sucrose, glucose, galactose, fructose, ribose, mannose, arabinose, and xylose solutions; the salt solution comprises phosphate-buffered saline, normal saline, borate buffer, Tris-HCl buffer, and HEPES buffer.

A responsive extracellular vesicle hydrogel, comprising extracellular vesicles and the polypeptide hydrogel, wherein the extracellular vesicles are encapsulated within the polypeptide hydrogel, and the polypeptide hydrogel comprises an isotonic solution of the polypeptide having the sequence of KSLSLSLGPASLSLSLK and a salt solution.

As a preferred embodiment, a responsive extracellular vesicle hydrogel is provided, wherein the extracellular vesicles encapsulate a drug. Extracellular vesicles may directly serve as drugs to treat diseases, or may encapsulate drugs to serve as delivery carriers for drugs to treat diseases. The extracellular vesicle hydrogel can enhance the delivery efficiency as a drug carrier, improve the retention of extracellular vesicles at the administration site, and control the release of extracellular vesicles and drugs from the hydrogel, thereby improving bioavailability. Encapsulated drugs include small molecule drugs, polypeptides, proteins, and nucleic acid drugs.

Further disclosed is the use of the polypeptide in the manufacture of a polypeptide hydrogel. The hydrogel, whose structure is less affected by changes in physiological or pathological environments, provides broad applicability to different diseases and different administration sites.

Further disclosed is the use of the polypeptide or the polypeptide hydrogel in the manufacture of a drug carrier. Experimental results of the present disclosure demonstrate that the KPK polypeptide hydrogel exhibits good biocompatibility. It is observed that primary neurons attached to and grown on the KPK hydrogel exhibit morphology similar to that of neurons grown on a poly-L-lysine substrate, with no significant differences in the number of neurons, the length of neurites, or the number of neurite branches, suggesting that the hydrogel can serve as a universal drug carrier capable of prolonging drug retention. Encapsulated drugs include extracellular vesicles, lipid nanoparticles, lipoproteins, small molecule drugs, polypeptides, proteins, nucleic acid drugs, cell therapy drugs, *etc*.

Further disclosed is the use of the polypeptide or the polypeptide hydrogel in the manufacture of a responsive extracellular vesicle hydrogel. The extracellular vesicle hydrogel is formed by encapsulating extracellular vesicles within the polypeptide hydrogel, thereby improving the retention of extracellular vesicles at the administration site, controlling the release of extracellular vesicles from the hydrogel, and further improving the utilization efficiency of extracellular vesicles.

Further disclosed is the use of the responsive extracellular vesicle hydrogel in the manufacture of a medicament for treating dermatological diseases, including treatment of skin damage and aging, promotion of wound healing, prevention and treatment of hypertrophic scars, reduction of skin pigmentation, and treatment of acne and pimples; ophthalmic diseases such as dry eye syndrome and macular degeneration; resistance and prevention of chronic obstructive pulmonary disease, treatment of acute respiratory distress syndrome; Alzheimer's disease in neurological disorders, neuronal cell repair in stroke, traumatic brain injury; promotion of endometrial repair in gynecological diseases, premature ovarian insufficiency, and acceleration of vaginal surgical wound repair; hepatic diseases such as hepatic fibrosis; chronic kidney disease; graft-versus-host disease in organ transplantation; osteoarthritis; and treatment of Alzheimer's disease of periodontitis in oral diseases, neuronal cell repair in stroke, glioma, mucosal injury repair, diabetes, and macular degeneration. Extracellular vesicles may be used in the treatment of Alzheimer's disease, neuronal cell repair in stroke, glioma, mucosal injury repair, diabetes, and ophthalmic diseases such as macular degeneration. The responsive extracellular vesicle hydrogel improves the retention of extracellular vesicles at the administration site, for example in the treatment of various ophthalmic diseases including traumatic diseases, autoimmune diseases, neovascular diseases, and glaucoma, thereby increasing their retention in the eye, suggesting that the extracellular vesicle hydrogel may be used at mucosal sites and the like to improve the retention and utilization efficiency of extracellular vesicles; enhance the accumulation of extracellular vesicles at glioma sites; prolongs the duration of action of extracellular vesicles at mucosal and skin injury sites, such as the ear canal and vagina. By controlling the release of extracellular vesicles from the hydrogel, the utilization efficiency of extracellular vesicles can be improved, resulting in more significant therapeutic effects for diseases treatable by extracellular vesicles.

As used herein, the term "hydrogel" refers to a hydrophilic three-dimensional structure with a certain viscosity, formed by the swelling of natural or synthetic polymer materials in water through chemical or physical crosslinking. Hydrogels formed from proteins or polypeptides exhibit good biocompatibility. Polypeptides are typically prepared through self-assembly and can spontaneously aggregate into nanofibers with highly ordered internal structures under specific conditions through intermolecular non-covalent interactions, such as ionic bonds, hydrophobic interactions, hydrogen bonds, and van der Waals forces, thereby forming hydrogels.

In a preferred embodiment of the present disclosure, the polypeptide hydrogel is prepared by dissolving the KPK polypeptide in an aqueous sucrose or mannitol solution to form a sucrose or mannitol solution of the KPK polypeptide. After the polypeptide is fully dissolved to form a clear and viscous solution, the pH is adjusted to neutral, and the solution is mixed with an equal volume of phosphate-buffered saline or normal saline to form a transparent KPK polypeptide hydrogel. In the present disclosure, the polypeptide hydrogel is prepared by mixing a sucrose solution of the KPK polypeptide with phosphate-buffered saline, or by mixing a mannitol solution of the KPK polypeptide with normal saline, or may be prepared by other conventional methods.

In a preferred embodiment of the present disclosure, the extracellular vesicle hydrogel is prepared by uniformly vortex-mixing the KPK polypeptide hydrogel with mesenchymal stem cell-derived extracellular vesicles (MSC-EVs) to form MSC-EVs-GEL, or may be prepared by other conventional methods.

As used herein, the terms "polypeptide hydrogel", "KPK polypeptide hydrogel", "KPK polypeptide gel", "KPK gel", and "KPK hydrogel" all refer to the KPK polypeptide-containing hydrogel formed by mixing an isotonic solution of the KPK polypeptide with a salt solution.

As used herein, the terms "MSC-EVs-GEL", "MSC-EVs gel", "MSC-EVs hydrogel", "MSC-EVs polypeptide gel", "MSC-EVs polypeptide hydrogel", and "extracellular vesicle hydrogel" all refer to the KPK polypeptide hydrogel encapsulating extracellular vesicles.

The advantage of the present disclosure is that the polypeptide designed in the present disclosure is prepared into an EV-responsive polypeptide hydrogel, which encapsulates extracellular vesicles to form an extracellular vesicle hydrogel, thereby improving the retention of extracellular vesicles at the administration site, controlling the release of extracellular vesicles from the hydrogel, and further improving the utilization efficiency of extracellular vesicles. The polypeptide hydrogel exhibits good biocompatibility, and the structure is less affected by changes in physiological or pathological environments. The polypeptide hydrogel provides broad applicability to different diseases and different administration sites, promotes the clinical translation and application of extracellular vesicles, and is also expected to serve as a universal drug carrier for improving the utilization efficiency of delivered drugs.

### BRIEF DESCRIPTION OF THE DRAWINGS

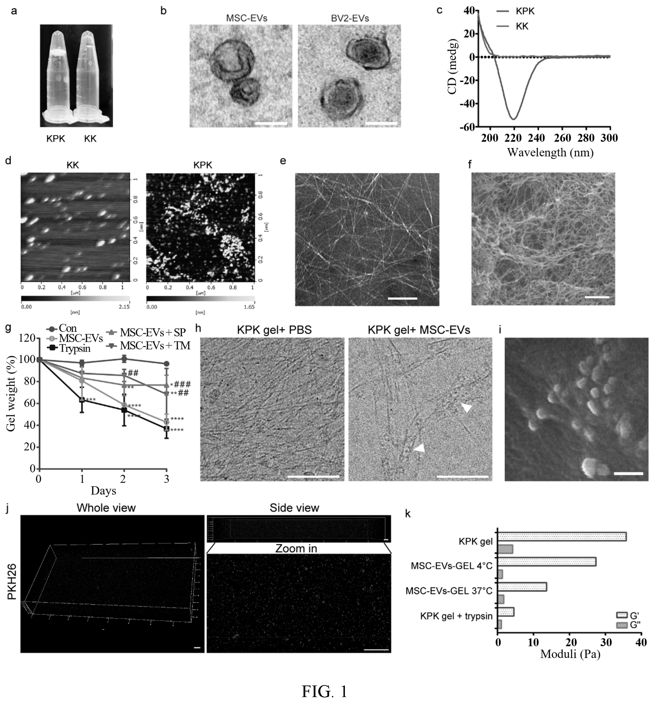

FIG. 1: Construction and characterization of MSC-EVs-GEL. (a) Morphology of KPK and KK hydrogels. (b) Electron microscopy images of MSC-EVs and BV2-EVs. Scale bar: 100 nm. (c) Circular dichroism spectroscopy results of KPK and KK hydrogels. (d) Atomic force microscopy (AFM) images of KPK and KK hydrogels. (e) Transmission electron microscopy (TEM) image of KPK hydrogel. Scale bar: 200 nm. (f) Scanning electron microscopy (SEM) image of KPK hydrogel. (g) Degradation of KPK hydrogel by MSC-EVs. The KPK hydrogel was incubated with PBS (Con), MSC-EVs solution (MSC-EVs), MSC-EVs solution containing 20 µM SP-13786 (MSC-EVs+SP), MSC-EVs solution containing 20 µM talabostat mesylate (MSC-EVs+TM), or trypsin at 37°C for 3 days. The remaining weight of the KPK hydrogel was monitored daily. (h) Cryo-TEM observation of the degradation of KPK hydrogel by MSC-EVs. The KPK hydrogel was incubated with PBS or 0.5 mg/mL MSC-EVs at 37°C for 48 hours. The white arrows indicate punctate morphology of the degraded KPK hydrogel. Scale bar: 100 nm. (i) Morphology of MSC-EVs-GEL observed by scanning electron microscopy. Scale bar: 100 nm. (j) Distribution of PKH26 (red)-labeled MSC-EVs in MSC-EVs-GEL observed by fluorescence confocal microscopy. Scale bar: 10 µm. (k) Degradation of MSC-EVs-GEL measured by rheometry. G': elastic modulus; G'': viscous modulus.

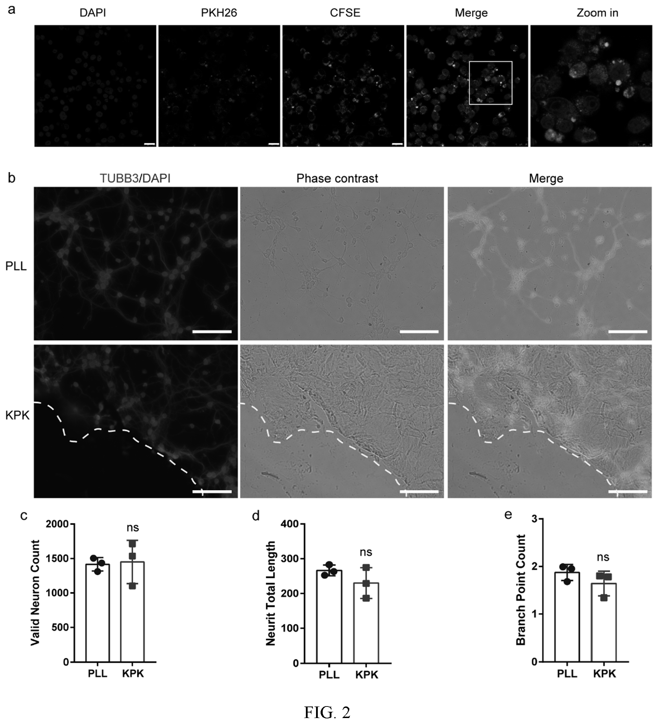

FIG. 2: MSC-EVs can be released from KPK hydrogel and promote neuronal growth. (a) Uptake by BV2 cells of MSC-EVs released from MSC-EVs-GEL observed by fluorescence confocal microscopy. MSC-EVs-GEL was loaded into a Transwell insert (1 µm pore size) and incubated with BV2 cells seeded in a 24-well plate for 24 hours. The membrane of MSC-EVs was labeled with PKH26 (red). The cargo of MSC-EVs was labeled with CFSE (green). DAPI (blue) indicates cell nuclei. Scale bar: 25 µm. (b) Morphological observation of the effect of KPK hydrogel on neuronal growth. Morphology of primary mouse neurons grown on poly-L-lysine (PLL)-coated or KPK hydrogel (KPK) for 6 days. Neurites were labeled with TUBB3 (red). Cell nuclei were labeled with DAPI (blue). Scale bar: 200 µm. The number of neurons (c), the total length of neurites (d), and the number of branch points (e) were analyzed using the high-content Neuronal Profiling program; n = 3.

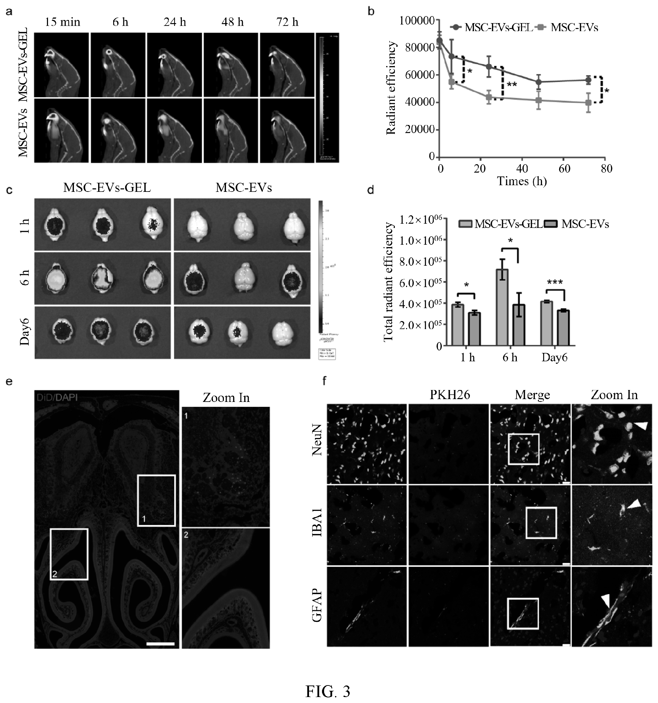

FIG. 3: MSC-EVs-GEL enhances nasal retention and brain delivery of MSC-EVs following intranasal administration. Mice were intranasally administered DiD-labeled MSC-EVs or MSC-EVs-GEL and observed using *in vivo* CT and fluorescence imaging. (a) Nasal retention. (b) Semi-quantitative analysis of nasal retention. (c) Brain distribution of MSC-EVs and MSC-EVs-GEL following intranasal administration. Mice were intranasally administered DiD-labeled MSC-EVs or MSC-EVs-GEL. Brain tissues were collected at 1 hour, 6 hours, and 6 days following administration and imaged using an *in vivo* fluorescence imaging system. (d) Semi-quantitative analysis of brain distribution of MSC-EVs and MSC-EVs-GEL following intranasal administration. (e) Distribution of DiD-labeled MSC-EVs (red) in the nasal cavity and olfactory bulb of mice after intranasal administration of MSC-EVs-GEL for 6 days. Scale bar: 50 µm. (f) Distribution of DiD-labeled MSC-EVs (red) in the cortex of mice after intranasal administration of MSC-EVs-GEL for 6 days. Astrocytes, microglia, and neurons were labeled with anti-GFAP antibody, anti-IBA1 antibody, and anti-NeuN antibody (green), respectively. Scale bar: 25 µm. Data are presented as mean ± standard deviation; n = 3.

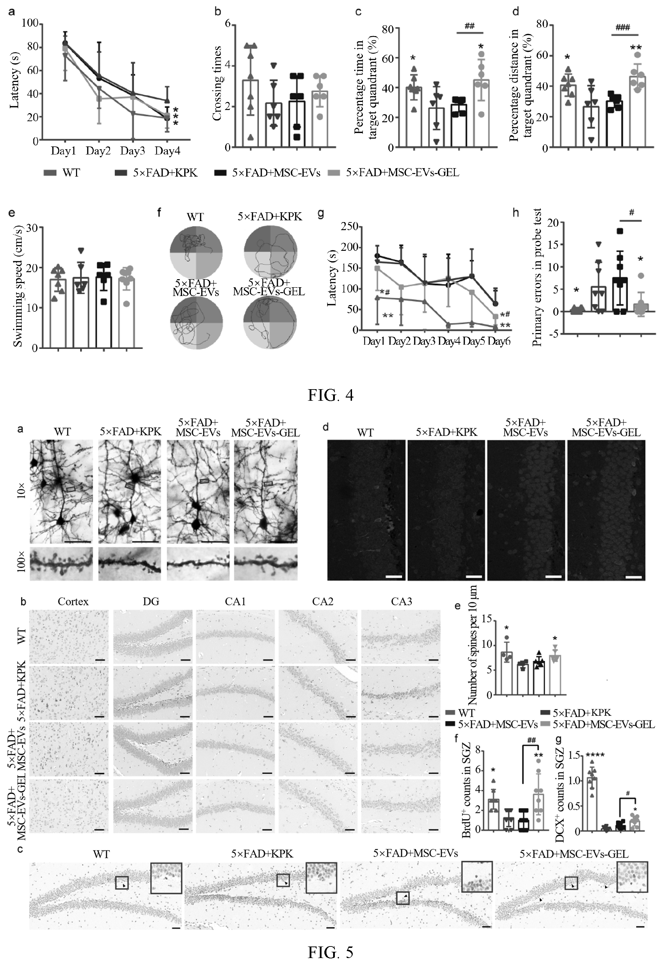

FIG. 4: MSC-EVs-GEL more effectively rescues memory dysfunction in 5×FAD AD model mice. 5×FAD mice were intranasally administered blank KPK hydrogel (5×FAD+KPK), MSC-EVs (5×FAD+MSC-EVs), or MSC-EVs-GEL (5×FAD+MSC-EVs-GEL), while wild-type littermate mice served as controls. Administration was performed once weekly for 5 weeks, followed by Morris water maze (MWM) testing and Barnes maze testing at week 6. (a to f) Results of the MWM test: (a) Latency in the navigation test; (b) Number of platform crossings in the probe trial; (c) Percentage of swimming time in the platform quadrant during the probe trial; (d) Percentage of swimming distance in the platform quadrant during the probe trial; (e) Swimming speed during the probe trial; (f) Representative swimming trajectories during the probe trial. Data are presented as mean ± standard deviation; n = 6 to 7. (g) Latency during acquisition training; (h) Number of visits to incorrect holes during the test trial. Data are presented as mean ± standard deviation; n= 7 to 8.
FIG. 5: MSC-EVs-GEL more effectively alleviates neuronal damage and promotes neurogenesis in 5×FAD AD model mice. (a) Golgi staining of cortical neurons in 5×FAD mice after intranasal administration of blank KPK hydrogel, MSC-EVs, or MSC-EVs-GEL for 5 weeks. Scale bar: 50 µm. (b) Nissl staining of the cortex, dentate gyrus, and hippocampal CA1, CA2, and CA3. Scale bar: 50 µm. (c) BrdU staining of the subgranular zone (SGZ) in 5×FAD mice and quantitative results (e). Scale bar: 25 µm. (d) Proliferation of neural stem cells and neurogenesis in the subgranular zone of the dentate gyrus in mice. (e) Quantitative analysis of Golgi staining of cortical neurons. (f) Quantitative analysis of BrdU staining of the subgranular zone (SGZ) in 5×FAD mice. (g) Quantitative analysis of proliferation of neural stem cells and neurogenesis in the subgranular zone of the dentate gyrus in mice using ImageJ. Scale bar: 40 µm.

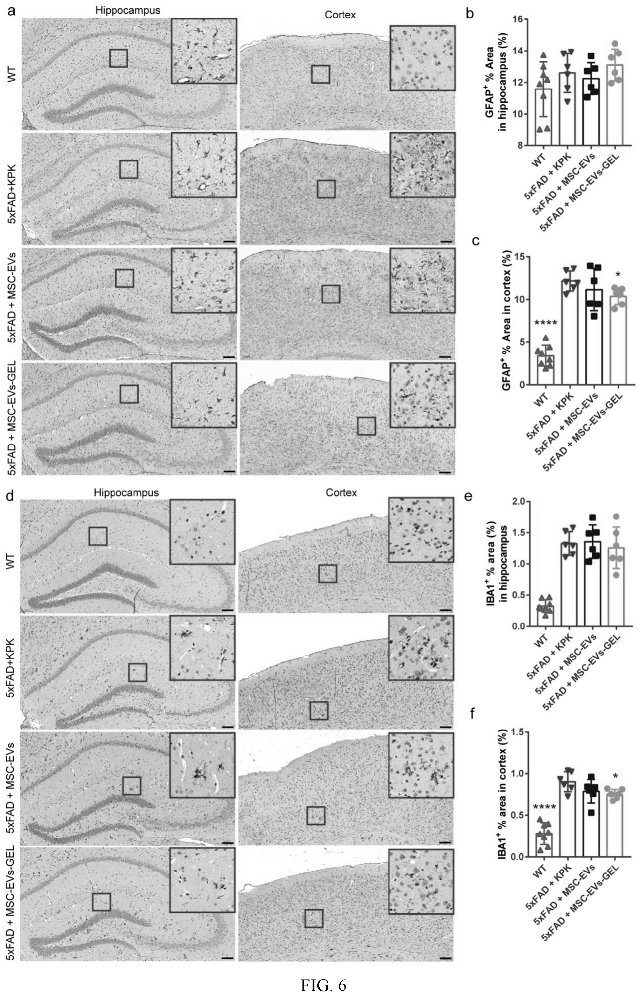

FIG. 6: Effect of MSC-EVs-GEL on astrocytes and microglia in 5×FAD mice. After 5 weeks of intranasal administration of KPK hydrogel, MSC-EVs, and MSC-EVs-GEL respectively to 5×FAD and WT mice: (a to c) GFAP immunostaining showing astrocytes in the mouse brain. (a) Morphology of astrocytes in the hippocampus and cortex of the mouse brain; Quantitative analysis of GFAP-positive staining area in the hippocampus (b) and cortex (c). (d to e) Effect of MSC-EVs-GEL on microglia in 5×FAD mice. IBA1 immunostaining showing microglia in the mouse brain. (d) Morphology of microglia in the hippocampus and cortex of the mouse brain; Quantitative analysis of IBA1-positive staining area in the hippocampus (e) and cortex (f). n = 6 to 8; Scale bar: 100 µm.

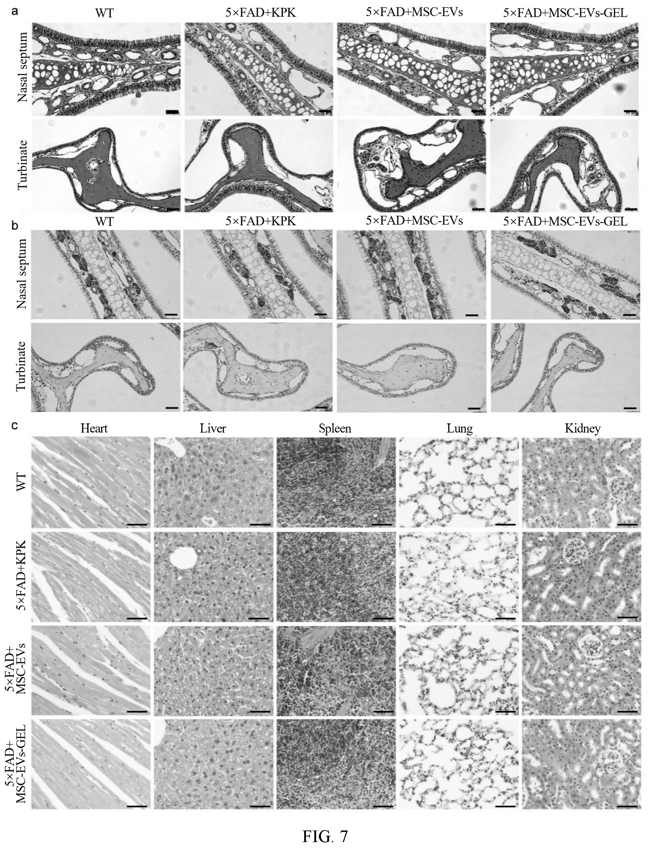

FIG. 7: MSC-EVs-GEL demonstrates good safety profile after 5 weeks of intranasal administration. (a to b) Morphology of the nasal mucosa in 5×FAD mice. (a) Morphology of the nasal septum and turbinate by HE staining; (b) Expression of neuron-specific enolase. Scale bar: 50 µm. (c) HE staining of major organs from 5×FAD and WT mice following treatment. Scale bar: 50 µm.

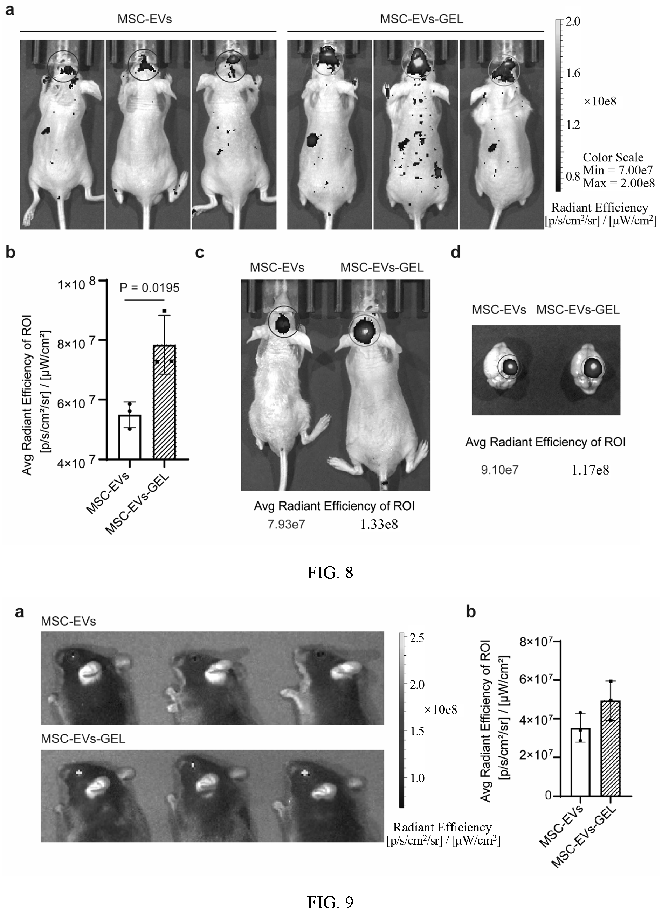

FIG. 8: Polypeptide hydrogel enhances accumulation of EVs at glioma sites. *In vivo* imaging (a) and quantitative data (b) after intranasal administration of DiO-labeled MSC-EVs-GEL to glioma model mice bearing orthotopically implanted human malignant glioblastoma cells (U87MG cell line) for 5 hours. *In vivo* imaging (c) and imaging of excised brain tissues (d) after intracranial administration of DiO-labeled MSC-EVs-GEL to orthotopic glioma model mice for 5 hours.
FIG. 9: Polypeptide hydrogel increases retention of MSC-EVs in the eye. *In vivo* imaging (a) and quantitative data (b) after topical ocular administration of DiO-labeled MSC-EVs-GEL to C57 mice for 4 hours.

### DETAILED DESCRIPTION OF THE PREFERRED EMBODIMENT

The technical solutions of the present disclosure are described in detail below with reference to specific embodiments. It should be understood that the following specific embodiments are provided solely to assist those skilled in the art in understanding the present disclosure and are not intended to limit the present disclosure.

### Example 1: Synthesis of polypeptides

In the present disclosure, the KPK polypeptide sequence (SEQ ID NO: 1, KSLSLSLGPASLSLSLK) was synthesized using solid-phase synthesis, and the non-responsive KK peptide (SEQ ID NO: 2, KSLSLSLSLSLSLK) was used as a negative control. The polypeptides were synthesized using solid-phase synthesis or liquid-phase synthesis, with a purity greater than 95%. The specific method for solid-phase synthesis was as follows: Amino acids were coupled onto chloromethyl polystyrene resin, and the amino protecting groups were removed in the presence of trifluoroacetic acid. The peptides were then cleaved with hydrogen fluoride, precipitated with ether in an ice bath, dissolved in acetonitrile, concentrated by rotary evaporation, and further purified by preparative high-performance liquid chromatography with an acetonitrile-water system.

### Example 2: Preparation of smart responsive release polypeptide hydrogel

The KPK polypeptide was dissolved in 298 mM aqueous sucrose solution filtered through a 0.22 µm filter to prepare a sucrose solution of the KPK polypeptide at a concentration of 2 to 200 mg/mL. The solution was allowed to swell at room temperature until the polypeptide was fully dissolved, forming a clear and viscous solution. The pH was adjusted to 7 using 1 N NaOH and checked using pH test paper. A certain volume of sucrose solution of the KPK polypeptide was mixed with an equal volume of PBS to form a transparent KPK hydrogel (FIG. 1a).

The KK polypeptide was dissolved in 298 mM aqueous sucrose solution filtered through a 0.22 µm filter to prepare a sucrose solution of the KK polypeptide at a concentration of 2 to 200 mg/mL. After dissolution, a clear but non-viscous aqueous solution was formed. The pH was adjusted to 7 using 1 N NaOH and checked using pH test paper. A certain volume of sucrose solution of the KK polypeptide was mixed with an equal volume of PBS, and the resulting mixture remained a clear solution without forming a hydrogel (FIG. 1a).

Construction of MSC-EVs-GEL: The above-prepared KPK hydrogel was uniformly vortex-mixed with MSC-EVs prepared by ultracentrifugation, polyethylene glycol precipitation, or an extracellular vesicle isolation kit (Capturem^{™} Extracellular Vesicle Isolation Kit (Code No. 635741), Takara) to form MSC-EVs-GEL, thereby encapsulating the extracellular vesicles within the polypeptide hydrogel. The resulting hydrogel was stored at 4°C.

### Example 3: Characterization of smart responsive release polypeptide hydrogel

MSC-EVs were prepared by ultracentrifugation. Particle size distribution was measured by dynamic light scattering. MSC-EVs exhibited a particle size of 114.0 ± 10.9 nm and a zeta potential of -41.4 ± 1.9 mV; while BV2-EVs exhibited a particle size of 128.4 ± 9.2 nm and a zeta potential of -42.3 ± 1.5 mV. Morphological observation by TEM showed that both MSC-EVs and BV2-EVs exhibited a characteristic cup-shaped morphology with a particle size of approximately 100 nm (FIG. 1b).

The secondary structures of the self-assembled hydrogels were analyzed by circular dichroism spectroscopy (FIG. 1c), wherein the KPK hydrogel exhibited a typical β-sheet conformation, while the KK sequence did not. AFM analysis on the nanostructures of the peptides after mixing with PBS showed that the KK peptide aggregated into particles with a size of approximately 1 nm but did not assemble into fibers (FIG. 1d), while the KPK peptide self-assembled into long and coiled fibrous structures with a diameter of approximately 1.65 nm. TEM and SEM results confirmed that KPK was able to self-assemble into elongated fibers, which further crosslinked to form a three-dimensional network structure (FIGs. 1e and 1f).

To determine the degradation characteristics of the KPK hydrogel in the presence of MSC-EVs, the KPK hydrogel was co-incubated with MSC-EVs, and the wet weight of the hydrogel was monitored daily. No significant weight loss was observed in the KPK hydrogel by the negative control PBS, whereas the positive control trypsin induced significant weight loss at all tested time points (FIG. 1g). Cryo-TEM analysis further confirmed the degradation of the KPK hydrogel after treatment with MSC-EVs (FIG. 1h). After co-incubation with MSC-EVs at 37°C for 48 hours, some KPK fibers disassembled and degraded into circular fragments (as indicated by the arrows in FIG. 1h). These results demonstrate that MSC-EVs are capable of degrading the KPK hydrogel.

MSC-EVs in PBS was added to an equal volume of 20 mg/mL KPK sucrose solution to prepare MSC-EVs-GEL. SEM observation of the microstructure of MSC-EVs-GEL showed that MSC-EVs were incorporated into the KPK hydrogel without causing morphological damage (FIG. 1i). Confocal microscopy analysis showed that PKH26-labeled MSC-EVs were uniformly dispersed in the KPK hydrogel as punctate signals without significant aggregation (FIG. 1j). The storage modulus G' and loss modulus G" of the KPK hydrogel and MSC-EVs-GEL were determined using oscillatory stress sweep analysis. The results showed that, after incubation at 4°C for 24 hours, MSC-EVs-GEL exhibited a G' value of 27 Pa, which was slightly lower than the G' value of the KPK hydrogel (36 Pa); after incubation at 37°C for the same duration, the G' value of MSC-EVs-GEL significantly decreased to 14 Pa (FIG. 1k). These results demonstrate that MSC-EVs can disrupt the structural integrity of MSC-EVs-GEL and induce changes in the rheological properties of the KPK hydrogel, and that such degradation activity is temperature-dependent.

A Transwell assay was further applied to investigate whether MSC-EVs released from MSC-EVs-GEL could be taken up by brain cells. BV2 microglial cells were used as a cell model. The membrane and cargo of MSC-EVs were labeled with PKH26 and carboxyfluorescein succinimidyl ester (CFSE), respectively. MSC-EVs-GEL was loaded into a Transwell insert, and BV2 cells were cultured at the bottom of a 24-well plate. After co-incubation for 24 hours, fluorescence imaging analysis showed that MSC-EVs released from MSC-EVs-GEL were successfully taken up by BV2 cells (FIG. 2a). Furthermore, co-localization of PKH26 and CFSE signals was observed within the cells, indicating that MSC-EVs were released from MSC-EVs-GEL and internalized by BV2 cells in an intact form.

Meanwhile, the KPK hydrogel exhibited good biocompatibility, as primary neurons attached to and grown on the KPK hydrogel exhibited morphology similar to that of neurons grown on a poly-L-lysine substrate (FIG. 2b), with no significant differences in the number of neurons, the length of neurites, or the number of neurite branches (FIG. 2c to 2e). Furthermore, as shown in FIG. 2b, neurites exhibited a distinct boundary at the interface between the KPK hydrogel and the bottom of the well plate, as primary neurons were unable to adhere to and grow on the uncoated regions at the bottom of the well plate, suggesting that the KPK hydrogel has the potential to serve as a substrate for promoting neuronal adhesion and growth. Addition of nerve growth factors to the KPK hydrogel significantly promoted the increase in the length of neurites and the number of neurite branches, suggesting that the KPK hydrogel can serve as a universal drug carrier.

### Example 4: MSC-EVs-GEL enhances nasal retention and brain delivery of MSC-EVs following intranasal administration

Mucociliary clearance results in a half-life of only 15 to 20 minutes for liquid formulations in the nasal cavity. To determine the retention of the MSC-EVs-GEL in the nasal cavity, MSC-EVs were labeled with DiD, and nude mice were intranasally administered MSC-EVs-GEL or MSC-EVs solution. CT/fluorescence images of the nasal cavity were collected at different time points following administration. The results showed that the retention of MSC-EVs-GEL in the nasal cavity was significantly higher than that of MSC-EVs solution at all time points following treatment (FIG. 3a). MSC-EVs solution exhibited a significantly higher clearance compared to MSC-EVs-GEL, particularly during the first 6 hours (FIG. 3b).

Brain delivery and transport pathways of MSC-EVs were further examined at 1 hour, 6 hours, and 6 days following administration. Treatment with MSC-EVs-GEL significantly increased brain delivery of MSC-EVs compared to MSC-EVs solution (FIG. 3c to 3d). Even 6 days after intranasal administration of MSC-EVs-GEL, strong fluorescence signals of MSC-EVs were still observed near the basal layer of the nasal mucosal epithelium (FIG. 3e). Fluorescence signals of MSC-EVs were also observed in the olfactory bulb region near the nasal mucosa. In the cortex, MSC-EVs were predominantly co-localized with neurons but sparsely distributed in microglial cells (FIG. 3f). A small amount of DiD fluorescence was observed near astrocytes at the margins of cerebral blood vessels (FIG. 3f), suggesting that MSC-EVs may be cleared from the brain via a perivascular pathway.

### Example 5: MSC-EVs-GEL more effectively improves cognitive dysfunction in 5×FAD AD model mice

Alzheimer's disease (AD) is the most common neurodegenerative disease and the leading cause of dementia. In our previous studies, intranasal administration of MSC-EVs to AD model mice every two days effectively improved cognitive dysfunction associated with AD. MSC-derived extracellular vesicles primarily function to promote angiogenesis, inhibit inflammation, prevent apoptosis, and promote tissue regeneration, while also promoting axonal regeneration and neurogenesis in the nervous system. Therefore, MSC-derived extracellular vesicles have high potential for the treatment of neurological disorders. The aforementioned results showed that, compared to MSC-EVs solution, MSC-EVs-GEL exhibited much longer retention time in the nasal cavity, and brain delivery of MSC-EVs could persist for at least 6 days after administration. Accordingly, MSC-EVs-GEL was intranasally administered once weekly for 5 weeks to the classic transgenic mouse model of AD, 5×FAD mice. Following administration, Morris water maze testing was performed to evaluate spatial learning and memory abilities of the animals. On day 4 of the navigation test, 5×FAD mice treated with MSC-EVs-GEL exhibited significantly reduced latency compared to those treated with blank KPK hydrogel (FIG. 4a). In the probe trial, although swimming speeds among different groups were similar, 5×FAD mice treated with MSC-EVs-GEL exhibited a significantly greater percentage of time and distance in the target quadrant compared to those treated with blank KPK hydrogel or MSC-EVs (FIGs. 4b to 4f).

Barnes maze testing was further performed to evaluate spatial learning and memory abilities in a dry environment. During the six consecutive days of acquisition training, latency decreased in all groups of mice. Compared to mice treated with blank KPK hydrogel, those treated with MSC-EVs-GEL exhibited significantly reduced latency in reaching the escape hole and significantly fewer visits to incorrect holes before reaching the escape hole (FIGs. 4g to 4h). Most importantly, model mice treated with MSC-EVs-GEL exhibited significantly fewer visits to incorrect holes in the probe trial compared to those treated with MSC-EVs solution (FIG. 4h). These results demonstrate that MSC-EVs-GEL effectively enhances the effect of MSC-EVs in improving spatial learning and memory abilities in 5×FAD mice.

### Example 6: MSC-EVs-GEL more effectively alleviates neuronal damage and promotes neurogenesis

Synaptic plasticity refers to changes in the number, morphology, and function of synapses resulting from prolonged neuronal activity, which forms the basis of learning and memory and is essential for brain function. A reduction in the number of synapses and changes in their plasticity are important pathological features of AD. To evaluate the effect of MSC-EVs-GEL on synaptic alterations, blank KPK hydrogel, MSC-EVs, and MSC-EVs-GEL were intranasally administered to 5×FAD and WT mice for 5 weeks, followed by Golgi staining of neuronal dendritic spines in the mouse brain. The results showed that, compared to WT mice, 5×FAD mice exhibited a significant reduction in dendritic spine density of cortical neurons, which was substantially restored after treatment with MSC-EVs-GEL (increased by 35.3%) (FIGs. 5a and 5e), suggesting that MSC-EVs-GEL may improve cognitive function by affecting dendritic spine density. Furthermore, Nissl staining was performed to evaluate the effect of MSC-EVs-GEL on the morphology of neuronal cell bodies (FIG. 5b). Nissl bodies are neuronal structures that reflect neuronal activity and function. Damaged neuronal cell bodies exhibit dark staining and shrinkage, leaving spaces between neurons, while normal neurons exhibit light blue staining and are arranged closely together. The results showed that, in model mice treated with MSC-EVs-GEL, Nissl bodies in the cortex, dentate gyrus, and hippocampal CA2 and CA3 were significantly more orderly and more lightly stained than those in mice treated with blank KPK hydrogel, with neuronal morphology and intercellular spaces being restored.

Newborn neurons exhibit significant plasticity that can improve learning and cognitive function. Therefore, the effect of MSC-EVs-GEL on the number of newborn neurons in the subgranular zone of the dentate gyrus in 5×FAD mice was further investigated. BrdU and doublecortin (DCX) served as markers for cell proliferation and immature neurons, respectively (FIG. 5c and 5f). MSC-EVs-GEL increased BrdU-positive cells by 262% and DCX-positive area by 369% compared to blank KPK hydrogel (FIG. 5d and 5g). Moreover, MSC-EVs-GEL exhibited stronger neurogenic effects than MSC-EVs liquid formulation, with 2.90-fold and 1.84-fold increases in BrdU-positive cells and DCX-positive area, respectively (FIG. 5f and 5g).

In addition to neuroprotection and neurogenesis, MSC-EVs have been shown to affect inflammatory responses. Activation of astrocytes in AD, also known as reactive gliosis, is a common phenomenon in the pathological process of AD, manifested by astrocyte proliferation and enhanced GFAP expression. GFAP staining results showed that, compared to 5×FAD mice treated with KPK, 5×FAD mice treated with MSC-EVs-GEL exhibited a 14.7% reduction in GFAP-positive area in the cortex (FIGs. 6a to 6c), suggesting that MSC-EVs-GEL exerts a certain inhibitory effect on the proliferation and activation of astrocytes in the cortex. Microglia are intrinsic immune cells of the central nervous system. Activation of microglia plays an important role in neuronal damage associated with AD. Microglia were first labeled with IBA1 to investigate the effect of MSC-EVs-GEL treatment on the number of microglia in the brains of model mice. The results showed that administration of MSC-EVs-GEL and MSC-EVs had no significant effect on the number of microglia in the hippocampus of 5×FAD mice. However, compared to the KPK treatment group, the MSC-EVs-GEL treatment group exhibited a 16.5% reduction in the number of microglia in the cortex (FIGs. 6d to 6e).

### Example 7: Safety evaluation of polypeptide hydrogel

As long-term administration is required for the polypeptide hydrogel, safety is of critical importance. The KPK polypeptide was prepared as a 20 mg/mL sucrose solution. A certain volume of the KPK polypeptide solution was mixed with PBS to form a transparent KPK hydrogel. The above-prepared KPK hydrogel was mixed with MSC-EVs prepared by ultracentrifugation to prepare MSC-EVs-GEL. After 5 weeks of intranasal administration, major organs were collected and sectioned to preliminarily evaluate the safety of intranasal administration of the KPK hydrogel. HE staining analysis of the nasal cavity showed that the mucosa on both sides of the nasal septum and turbinate remained intact in all experimental groups (FIG. 7a), indicating good safety for nasal administration. Neuron-specific enolase antibody staining demonstrated that neurons in the nasal mucosa exhibited deeply stained continuous structures with uniform thickness (FIG. 7b). In the heart, liver, spleen, and kidney tissues of each treatment group, no abnormalities were observed in myocardial fibers; hepatocytes showed no cloudy swelling; the structures of white pulp and red pulp of the spleen were clear; the renal glomerular and tubular structures were normal, with no microscopically visible morphological changes (FIG. 7c).

### Example 8: Polypeptide hydrogel enhances accumulation of EVs at glioma sites

Glioma is a type of highly malignant primary intracranial tumors, ranking third among all tumors in five-year mortality rate. Due to the infiltrative growth characteristics of glioma cells, surgical resection is typically followed by adjuvant therapy. Although some novel therapeutics have been developed and approved, the heterogeneity and progression of tumors, combined with the limited efficiency of intracerebral delivery, urgently necessitate the development of therapeutics capable of efficiently entering the brain and intervening in glioma progression. Therefore, MSC-EVs-GEL, which can increase brain delivery via intranasal administration, has considerable potential for the treatment of glioma. The KPK polypeptide was prepared as a 20 mg/mL mannitol solution. A certain volume of the KPK polypeptide solution was mixed with normal saline to form a transparent KPK hydrogel, followed by addition and mixing of DiO-labeled MSC-EVs to prepare MSC-EVs-GEL. After intranasal administration of DiO-labeled MSC-EVs-GEL to glioma model mice bearing orthotopically implanted human malignant glioblastoma cells (U87MG cell line) for 5 hours, *in vivo* imaging showed that brain delivery of MSC-EVs-GEL was significantly higher than that of MSC-EVs (FIG. 8a and 8b). Meanwhile, after intracranial administration of MSC-EVs and MSC-EVs-GEL to orthotopic glioma model mice for 5 hours, the retention of MSC-EVs-GEL in the brain was 1.28-fold higher than that of MSC-EVs. These results demonstrate that the polypeptide hydrogel enhances brain delivery of MSC-EVs in glioma models.

### Example 9: Polypeptide hydrogel prolongs the duration of action of EVs in the eye

In the field of ophthalmology, extracellular vesicles may be used for the treatment of various ophthalmic diseases, including traumatic diseases, autoimmune diseases, neovascular diseases, and glaucoma. For example, extracellular vesicles may be used for delivering drugs or mRNAs that promote cell regeneration and reduce inflammation, and may also be used for delivering anti-angiogenic and neuroprotective agents. However, due to the flushing effect of tears, extracellular vesicles exhibit short residence time in the eye after administration, resulting in limited utilization efficiency. Therefore, extracellular vesicle hydrogel formulations may improve the retention of extracellular vesicles in the eye, thereby enhancing the bioavailability of EVs. After topical ocular administration of DiO-labeled MSC-EVs-GEL to C57 mice for 4 hours, *in vivo* imaging showed that the retention of MSC-EVs-GEL in the eye was significantly higher than that of MSC-EVs (FIG. 9), suggesting that MSC-EVs-GEL can improve the bioavailability of MSC-EVs in the eye.

### Example 10: Polypeptide hydrogel prolongs the duration of action of EVs in the ear canal, vagina, and skin injury sites

The aforementioned results showed that the hydrogel formulation prolonged the residence time of EVs in the eye, suggesting that the hydrogel may be used at mucosal sites and the like to improve the retention and utilization efficiency of EVs. A mouse model of secretory otitis media was established by intraperitoneal injection of ovalbumin combined with the immune adjuvant aluminum oxide. After administration of MSC-EVs-GEL into the ear canal for 5 hours, the retention of MSC-EVs-GEL in the injured ear canal was significantly higher than that of MSC-EVs, being 3.4-fold that of the MSC-EVs group.

Preparation of vaginitis model: 10-week-old female ICR mice were subcutaneously injected every two days with 0.05 mL of oil solution containing 0.1 mg estradiol benzoate for a total of three injections, followed by intravaginal inoculation with 20 µL of Candida albicans suspension (containing approximately 50,000 Candida albicans spores). Four days after model establishment, MSC-EVs-GEL was intravaginally administered for 5 hours, and the retention of MSC-EVs-GEL and MSC-EVs in the vagina was measured. The results showed that the retention of MSC-EVs-GEL was 2.85-fold that of the MSC-EVs group.

In a rat model of full-thickness skin defect wounds, after administration of MSC-EVs-GEL for 4 hours, the retention of MSC-EVs-GEL at the skin injury site was 2.3-fold that of the MSC-EVs group.

These results demonstrate that the hydrogel formulation prolongs the duration of action of EVs at various mucosal and skin sites.

The foregoing descriptions are merely preferred embodiments of the present disclosure. It should be noted that, for those of ordinary skill in the art, various improvements and modifications may be made without departing from the principles of the present disclosure, and such improvements and modifications shall also fall within the scope of protection of the present disclosure.

## Claims

1. A polypeptide, wherein the sequence of the polypeptide is KSLSLSLGPASLSLSLK.

2. A polypeptide hydrogel, wherein the polypeptide hydrogel is formed by mixing an isotonic solution of the polypeptide according to claim 1 and a salt solution.

3. The polypeptide hydrogel according to claim 2, wherein the isotonic solution comprises one or more of sucrose, glucose, galactose, fructose, ribose, mannose, arabinose, and xylose solutions, the salt solution comprises one or more of phosphate-buffered saline, normal saline, borate buffer, Tris(hydroxymethyl)aminomethane hydrochloride buffer solution, and 4-(2-Hydroxyethyl) piperazine-1-ethanesulfonic acid buffer solution.

4. A responsive extracellular vesicle hydrogel, wherein the responsive extracellular vesicle hydrogel comprises extracellular vesicles and the polypeptide hydrogel according to claim 2, the extracellular vesicles are encapsulated within the polypeptide hydrogel.

5. The responsive extracellular vesicle hydrogel according to claim 4, wherein the extracellular vesicles encapsulate a drug, the drug comprises small molecule drugs, polypeptides, proteins, and nucleic acid drugs.

6. Use of the polypeptide according to claim 1 in the manufacture of a polypeptide hydrogel.

7. Use of the polypeptide according to claim 1 or the polypeptide hydrogel according to claim 2 in the manufacture of a drug carrier.

8. Use of the polypeptide according to claim 1 or the polypeptide hydrogel according to claim 2 in the manufacture of a responsive extracellular vesicle hydrogel.

9. Use of the responsive extracellular vesicle hydrogel according to claim 4 or 5 in the manufacture of a medicament for the treatment of skin damage and aging, promotion of wound healing, prevention and treatment of hypertrophic scars, reduction of skin pigmentation, acne and pimples, dry eye syndrome, macular degeneration, resistance and prevention of chronic obstructive pulmonary disease, acute respiratory distress syndrome, Alzheimer's disease, neuronal repair after stroke, traumatic brain injury, promotion of endometrial repair, premature ovarian insufficiency, vaginal surgical wound repair, hepatic fibrosis, chronic kidney disease, graft-versus-host disease, osteoarthritis, and periodontitis.
